(19) Europäisches Patentamt
European Patent Office
Office européen des brevets

(11) Publication number: **0 368 130 B1**

# (12) EUROPEAN PATENT SPECIFICATION

(45) Date of publication of patent specification: **04.05.94** (51) Int. Cl.[5]: **A61K 7/16**

(21) Application number: **89120243.4**

(22) Date of filing: **02.11.89**

The file contains technical information submitted after the application was filed and not included in this specification

## (54) Oral compositions.

(30) Priority: **09.11.88 US 269227**

(43) Date of publication of application:
**16.05.90 Bulletin 90/20**

(45) Publication of the grant of the patent:
**04.05.94 Bulletin 94/18**

(84) Designated Contracting States:
**AT BE CH DE ES FR GB GR IT LI LU NL SE**

(56) References cited:
**EP-A- 0 026 252**
**EP-A- 0 173 568**
**EP-A- 0 177 303**
**DE-A- 3 415 147**
**DE-A- 3 541 025**
**US-A- 4 340 583**

(73) Proprietor: **THE PROCTER & GAMBLE COMPANY**
**One Procter & Gamble Plaza**
**Cincinnati Ohio 45202(US)**

(72) Inventor: **Burgess, Steven Carl**
**3517 Maple Avenue**
**Sharonville Ohio 45241(US)**
Inventor: **Guay, Christopher Barry**
**11943 Briarfield Court**
**Cincinnati Ohio 45240(US)**
Inventor: **Rebitski, George Forrest**
**9593 Colegate Way**
**Hamilton Ohio 45011(US)**

(74) Representative: **Brooks, Maxim Courtney et al**
**Procter & Gamble Limited**
**Whitley Road**
**Longbenton**
**Newcastle-upon-Tyne NE12 9TS (GB)**

EP 0 368 130 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to toothpaste compositions containing an organic, cationic therapeutic agent and a silica abrasive having excellent compatibility with said agent.

The use of antimicrobial agents to reduce plaque/gingivitis as well as mouth odor has been recognized for many years. Included among references disclosing oral compositions containing antimicrobials are US-A-3,937,805, February 10, 1976 to Harrison; US-A-3,937,807, February 10, 1976 to Haefele; US-A-4,080,441, March 21, 1978 to Gaffer et al.; US-A-4,118,474, October 3, 1978 to Gaffar et al.; US-A-4,241,049, December 23, 1980 to Colodney et al.; US-A-3,925,543, December 9, 1975 to Donohue; and US-A-4,256,731, March 17, 1981 to Curtis et al. Additionally, EP-A-0,181,161 discloses a fluoride free oral hygiene product containing a cationic bisbiguanide and zinc. Still another reference disclosing zinc plus organic, cationic therapeutic agents is US-A-4,022,880, May 10, 1977 to Vinson et al.

Particular problems faced when formulating dentifrices containing ionic therapeutic agents is ensuring the availability of the agents in the formulation. There are a number of conventional dentifrice components which will complex with such agents thereby making the agents not available to serve their function.

One approach which has been used to improve compatibility with therapeutic agents is to treat the silica with hydrofluoric acid. Such a disclosure is in US-A-3,862,307, January 21, 1975 to DiGuilio. Another patent disclosing dentifrice compositions containing organic, cationic therapeutic agents and a more compatible silica abrasive is US-A-4,157,387, June 5, 1979 to Benedict. The greater compatibility is obtained by coating the abrasive with a water soluble polymer. Still another patent disclosing silica abrasives having good compatibility with therapeutic agents is US-A-4,421,527, December 20, 1987 to Wason. The abrasives are obtained by treating the alkali metal silicate subsequent to its acidulation with an alkaline earth metal.

While there are disclosed in the prior art oral compositions containing antiplaque/antigingivitis agents and compatible abrasives there is still the need for better executions.

It is an object of the present invention to provide toothpaste compositions providing antiplaque/antigingivitis benefits.

It is a further object of the present invention to provide toothpaste compositions balanced to provide maximum availability of the active components.

It is still a further object of the present invention to provide toothpastes containing an organic, cationic therapeutic agent.

It is still a further object of the present invention to provide oral compositions containing zinc ions.

It is still a further object of the present invention to provide a method of reducing plaque/gingivitis through the use of the compositions described herein.

These and other objects will become readily apparent from the detailed description which follows.

All percentages and ratios used herein are by weight unless otherwise specified. Further, all measurements are made at 25°C unless otherwise specified.

According to the present invention there is provided a toothpaste composition consisting essentially of

(a) from 10% to 35% by weight of a precipitated silica abrasive having good compatibility with cationic therapeutic agents;

(b) from 0.25% to 2% by weight of a nonionic thickening agent selected from hydroxyethyl cellulose, hydroxypropyl cellulose, hydroxypropyl methyl cellulose, guar gum, locust bean gum and mixtures thereof;

(c) from 0.5% to 2% by weight of a nonionic surfactant selected from block copolymers of ethylene oxide and propylene oxide, POE sorbitan esters, sorbitan fatty esters, ethoxylated fatty acids, PEG esters and mixtures thereof;

(d) from 30% to 70% by weight of a nonionic humectant selected from sorbitol, propylene glycol, glycerine and mixtures thereof;

(e) from 0.1% to 5% by weight of an organic cationic, therapeutic agent selected from quaternary ammonium compounds, substituted guanidines and mixtures thereof; and

(f) water,

characterised in that the precipitated silica abrasive contains 10-300 parts per million of alkaline earth metal ion, and has an RDA value of at least 40, an oil absorption value of 70-100 ccs/100 gram, a pack density of between 0.24 and 0.055 $gcm^{-3}$, a loss on ignition value of between 4 to 6% and a BET surface area of 10 to 300 $m^2/g$ with an average particle size of 2 to 25 $\mu m$, a sulfate ion level below 0.25% and a pH in the range of 4.0-7.5.

Methods of reducing plaque/gingivitis with these compositions are also disclosed herein.

The necessary as well as optional components of the compositions of the present invention are described in detail hereinafter.

By "safe and effective" as used herein, means sufficient compound to reduce plaque/gingivitis while being safe to the hard and soft tissues of the oral cavity.

By the term "comprising", as used herein, is meant that various additional components can be conjointly employed in the compositions of this invention so long as they do not interfere with the therapeutic agents' ability to perform their intended function.

Silica Abrasive

The abrasive herein is provided by J. M. Huber Corporation. Such precipitated abrasives contain 10-300 parts per million of alkaline earth metal ions, and are characterized by an RDA value of at least 40, an oil absorption value of 70-100 ccs/100 gram, a pack density of between 0.24 and 0.055 $gmcm^{-3}$, a loss on ignition value of between 4 to 6% and a BET surface area of 10 to 300 $m^2/g$ with an average particle size of 2 to 15 µm; a sulfate ion level below 0.25% and a pH in the range of 4.0-7.5.

A method for producing the J. M. Huber experimental abrasive comprises forming an aqueous solution of an alkali metal silicate having an $SiO^2$ to $X_2O$ mole ratio of 2.0 to 3.3, wherein X is alkali metal, at a reaction temperature in the range of 77° to 91°C, acidulating with a mineral acid until precipitation of silicon dioxide is substantially complete, then continuing the mineral acid addition until the pH is 6.0 or less, digesting at a temperature 10-30°C higher than the reaction temperature for a period of 10-30 minutes, filtering the resulting slurry and washing the solid product with fresh water to reduce the sulfate ion concentration to below 0.25%, reslurrying the resulting wet cake in water and under agitation conditions, adding sufficient alkaline earth metal ion in the form of a sufficiently soluble alkaline earth metal salt or compound in sufficient amount to add to said wet cake alkaline earth metal ions in the range of 10-300 parts per million based on the dry recoverable product in said slurry, agitating the resulting mixture to provide adherence of the effective level of said alkaline earth metal treatment on the surface of said silicon dioxide, acidulating to reduce the pH below 5.5, drying and recovering the solid product.

The abrasive is present at a level of from 10% to 35%, preferably from 15% to 27%.

Nonionic Thickening Agents

The nonionic thickening agents useful herein are selected from hydroxyethyl cellulose, hydroxy propyl cellulose, hydroxy propyl methyl cellulose, guar gum, locust bean gum and mixtures thereof. The thickening agent is present at a level of from 0.25% to 2.0% preferably from 0.75% to 1.75%.

Nonionic Surfactant

The nonionic surfactants useful in the present compositions are selected from copolymers of ethylene oxide and propylene oxide, POE sorbitan esters, sorbitan fatty esters, ethoxylated fatty acids and PEG esters. A preferred surfactant is PEG(40) sorbitan diisostearate. The surfactant is used at a level of from 0.50% to 2.0%, preferably from 0.9% to 1.5%.

Nonionic Humectant

The humectants useful in the present compositions are selected from propylene glycol, glycerine, sorbitol and mixtures thereof. The humectant is present at a level of from 30% to 70%, preferably from 40% to 65%.

Organic, Cationic Therapeutic Agent

Organic cationic therapeutic agents suitable herein include quaternary ammonium compounds. Antiplaque, antigingivitis quaternary ammonium compounds include those in which one or two of the substituents on the quaternary nitrogen has a carbon chain length (typically alkyl group) of some 8 to 20, typically 10 to 18, carbon atoms while the remaining substituents (typically alkyl or benzyl group) have a lower number of carbon atoms, such as 1 to 7 carbon atoms, typically methyl or ethyl groups. Dodecyl trimethyl ammonium bromide, benzalkonium chloride, domiphen bromide dodecyl dimethyl (2-phenoxyethyl) ammonium bromide, benzyl dimethylstearyl ammonium chloride, cetyl pyridinium chloride and

quaternized 5-amino-1, 3-bis(2-ethyl-hexyl)-5-methyl hexa hydropyrimidine are exemplary of typical quaternary ammonium antibacterial agents.

Another antibacterial, antiplaque quaternary ammonium compound is benzethonium chloride, also known as Hyamine (RTM) 1622 or diisobutylphenoxyethoxyethyl dimethyl benzyl ammonium chloride. In an oral composition this material is highly effective in promoting oral hygiene by reducing formation of dental plaque, which is generally accompanied by a reduction in caries formation and periodontal diseases. Other cationic antibacterial agents of this type are those mentioned, for instance, in US-A-2,984,639, 3,325,402, 3,431,208 and 3,703,583 and GB-A-1,319,396.

Another organic, cationic therapeutic agent suitable for use in the present compositions are substituted guanidines. The substituted guanidines of this invention include bisbiguanide compounds having the generic formula:

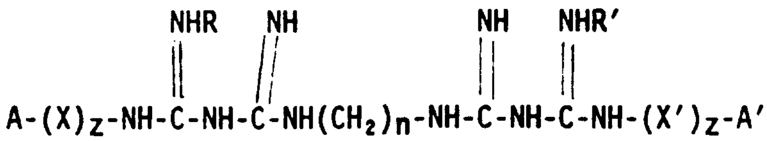

wherein A and A' each represent either (1) a phenyl radical which optionally is substituted by an alkyl or alkoxy group containing from 1 to 4 carbon atoms, a nitro group, or a halogen atom; (2) an alkyl group containing from 1 to 12 carbon atoms; or (3) alicyclic groups containing from 4 to 12 carbon atoms; wherein X and X' each represent an alkylene radical containing from 1 to 3 carbon atoms; wherein Z and Z' each can be either 0 or 1; wherein R and R' each represent either hydrogen, an alkyl radical containing from 1 to 12 carbon atoms, or an aralkyl radical containing from 7 to 12 carbon atoms; wherein n is an integer from 2 to 12 inclusive; wherein the polymethylene chain $(CH_2)_n$ may optionally be interrupted by oxygen or sulfur atoms, aromatic nuclei, etc. The water soluble salts of the above compounds are preferred for use herein. Suitable water soluble salts include the chloride, the fluoride, and especially the acetate salt. The preferred substituted guanidine is chlorhexidine-[1,6-di(-$N^5$-pchlorophenyl-N-diguanido)-hexane], particularly the digluconate salt.

The cationic antimicrobial is used in the present compositions at a level of from 0.1% to 5.0%, preferably from 0.3% to 1.5%, most preferably from 0.75% to 1.25%.

## Optional Components

Many other components may be included in the present compositions to make them more effective or aesthetically pleasing.

One such optional component is a fluoride ion source. It is common to have a water-soluble fluoride compound present in dentifrices in an amount sufficient to give a fluoride concentration of from 0.0025% to 5.0% by weight, preferably from 0.005% to 2.0% by weight, to provide additional anticaries effectiveness. Preferred fluorides are sodium fluoride, stannous fluoride, indium fluoride, and sodium monofluorophosphate. Norris et al., US-A-2,946,725, issued July 26, 1960 and Widder et al., US-A-3,678,154, issued July 18, 1972 disclose such salts as well as others. The present compositions also provide for high percentages of the fluoride ions to be available.

Another desirable optional component is a zinc ion source. Examples of zinc compounds that may be employed are zinc salts of the following organic and inorganic anions: acetate, benzoate, borate, bromide, carbonate, citrate, chloride, glycerophosphate, hexafluorosilicate, di-lactate (trihydrate), nitrate, phenolsulfonate, silicate, alkanoates having 8 to 18 carbon atoms, such as zinc stearate, salicylate, stannate, sulfate, tannate, titanate, tetrafluoroborate, oxide, peroxide, tartrate, etc. The zinc compounds may be used singly or in admixture.

The amount of zinc compound used should be sufficient to provide from 0.1% to 1.5% zinc ions, preferably from 0.2% to 0.5%.

The pH of the present compositions and/or its pH in the mouth should be from 4.5 to 7.5, preferably from 5.0 to 7.0.

Flavoring and sweetening agents may also be added to toothpaste compositions. Suitable flavoring agents include oil of wintergreen, oil of peppermint, oil of spearmint, oil of sassafras, and oil of clove. Sweetening agents which can be used include aspartame, acesulfame, saccharin, dextrose, levulose and sodium cyclamate. Flavoring and sweetening agents are generally used in dentifrices at levels of from 0.005% to 2% by weight.

Another optional component which may be useful in the present compositions in an antistain agent. As with other antimicrobials the materials used in the present compositions may cause staining when used at fairly high levels. Antistain agents include carboxylic acids such as those disclosed in US-A-4,256,731, May 17, 1981 to Curtis et al.. Other agents include amino carboxylate compounds as disclosed in US-A-3,937,807, February 10, 1976 to Haefele; dicarboxylic acid esters as disclosed in US-A-4,080,441, March 21, 1978 to Gaffar et al.; and phosphonoacetic acid as disclosed in US-A-4,118,474, October 3, 1978 to Gaffar et al. Many other agents in addition to those discussed herein may also be used. If used these agents are generally in an amount of 0.05% or greater. The antistain active may be used in the same composition with the cationic antimicrobial compound or in a separate composition used sequentially.

The toothpaste carrier compositions of the present invention may be made using methods such as those described In the Examples.

The following examples further describe and demonstrate the preferred embodiments within the scope of the present invention.

Unless otherwise indicated, all percentages herein are by weight.

EXAMPLES I - VI

The following are toothpastes representative of the present invention:

| Component | I | II | III | IV | V | VI |
|---|---|---|---|---|---|---|
| Sorbitol | 36.288% | 36.288% | 36.288% | 36.600% | 6.288% | 36.288% |
| Glycerin | 34.625% | 34.625% | 34.625% | 34.625% | 4.625% | 34.625% |
| Silica | 20.202% | 20.202% | 20.202% | 20.202% | 0.202% | 20.202% |
| PEG 40 SDIS[2] | 1.212% | 1.212% | -- | -- | 1.212% | 1.212% |
| Mackine (RTU) 101[2] | - | -- | 1.212% | -- | -- | -- |
| Pluronic (RTU) F127[2] | - | -- | -- | 0.900% | -- | -- |
| Modified Gleem Flavor | 0.808% | 0.808% | 0.808% | 0.808% | 0.808% | 0.808% |
| CH Gluconate (20% Solution) | 5.350% | 5.350% | 5.350% | 5.350% | 5.350% | 5.350% |
| Natrosol (RTU) 250M[1] | 1.515% | 1.515% | 1.515% | 1.515% | 1.515% | 1.515% |
| Product pH | 5.9 | 5.6 | 5.9 | 6.2 | 5.1 | 5.5 |

[1]Nonionic binder provided by Aqualon Inc. which is hydroxyethyl cellulose

[2]Nonionic surfactants

In preparing the dentifrice formulations, sorbitol and 70% of the glycerin are added to the mix tank and heating to 66 °C is initiated. Once the desired temperature has been attained, the silica is added under agitation and allowed to disperse. Sufficient agitation is maintained to prevent settling of the insoluble silica.

After the silica is thoroughly dispersed, the surfactant, flavor, and chlorhexidine are added and allowed to dissolve.

The binder system is added by first dispersing the binder (hydroxyethyl cellulose) in the remainder of the glycerin in a separate vessel. When the binder is well dispersed, the mixture is added to the main mix tank. The resulting paste is allowed to mix for 10 minutes.

Finally, the paste is milled and deaerated in the normal manner to remove any remaining lumps and entrapped air.

EXAMPLE VII

| Ingredients | %W/W |
|---|---|
| Sodium Fluoride | 0.320 |
| Zinc Lactate | 1.300 |
| Natroso (RTM) 250M | 1.200 |
| Glycerine | 16.400 |
| PEG 6 | 1.000 |
| Sorbitol 70% Solution | 37.310 |
| Huber CHX-Silica | 27.000 |
| Titanium dioxide | 0.700 |
| PEG 40 - SDIS | 1.200 |
| Acesulfame-K (RTM) | 0.375 |
| AZ15 Flavor Blend | 1.000 |
| Water | 10.000 |
| *Chlorhexidine digluconate (20% w/v solution) | 2.120 |
| FD&C Blue No. 1 (1%) | 0.075 |
| | 100.000 |

## Claims

**1.** A toothpaste composition consisting essentially of:

(a) from 10% to 35% by weight of a precipitated silica abrasive having good compatibility with cationic therapeutic agents;

(b) from 0.25% to 2% by weight of a nonionic thickening agent selected from hydroxyethyl cellulose, hydroxypropyl cellulose, hydroxypropyl methyl cellulose, guar gum, locust bean gum and mixtures thereof;

(c) from 0.5% to 2% by weight of a nonionic surfactant selected from block copolymers of ethylene oxide and propylene oxide, POE sorbitan esters, sorbitan fatty esters, ethoxylated fatty acids, PEG esters and mixtures thereof;

(d) from 30% to 70% by weight of a nonionic humectant selected from sorbitol, propylene glycol, glycerine and mixtures thereof;

(e) from 0.1% to 5% by weight of an organic cationic, therapeutic agent selected from quaternary ammonium compounds, substituted guanidines and mixtures thereof; and

(f) water,

characterised in that the precipitated silica abrasive contains 10-300 parts per million of alkaline earth metal ion, and has an RDA value of at least 40, an oil absorption value of 70-100 ccs/100 gram, a pack density of between 0.24 and 0.055 $gcm^{-3}$, a loss on ignition value of between 4 to 6% and a BET surface area of 10 to 300 $m^2/g$ with an average particle size of 2 to 25 m, a sulfate ion level below 0.25% and a pH in the range of 4.0-7.5.

**2.** A toothpaste composition according to Claim 1 wherein the cationic therapeutic agent is a substituted guanidine.

**3.** A toothpaste composition according to any of Claims 1-2 which in addition contains a zinc ion source.

**4.** A toothpaste composition according to any of Claims 1-3 wherein the surfactant is PEG(40) sorbitan diisostearate.

**5.** A toothpaste composition according to any of Claims 1-4 wherein the substituted biguanide is chlorhexidine digluconate.

6. A toothpaste composition according to Claim 3 wherein the zinc ion source is zinc lactate.

7. A toothpaste composition according to any of Claims 1-6 which in addition contains a fluoride ion source.

8. A toothpaste composition according to any of Claims 1-7 wherein the fluoride ion source is sodium fluoride and the thickening agent is hydroxyethyl cellulose.

**Patentansprüche**

1. Zahnpastenzusammensetzung, welche im wesentlichen aus:
   (a) 10 Gew.-% bis 35 Gew.-% von einem gefällten Siliciumdioxid als Schleifmittel, welches eine gute Verträglichkeit mit kationischen therapeutischen Mitteln aufweist;
   (b) 0,25 Gew.-% bis 2 Gew.-% von einem nichtionischen Verdickungsmittel, welches unter Hydroxyethylcellulose, Hydroxypropylcellulose, Hydroxypropylmethylcellulose, Guargummi, Johannisbrotkernmehl und Gemischen hievon ausgewählt ist;
   (c) 0,5 Gew.-% bis 2 Gew.-% von einem nichtionischen grenzflächenaktiven Mittel, welches unter Blockcopolymeren aus Ethylenoxid und Propylenoxid, POE-Sorbitanestern, Sorbitanfettestern, ethoxylierten Fettsäuren, PEG-Estern und Gemischen hievon ausgewählt ist;
   (d) 30 Gew.-% bis 70 Gew.-% von einem nichtionischen Feuchthaltemittel, welches unter Sorbit, Propylenglykol, Glycerin und Gemischen hievon ausgewählt ist;
   (e) 0,1 Gew.-% bis 5 Gew.-% von einem organischen kationischen, therapeutischen Mittel, welches unter quaternären Ammoniumverbindungen, substituierten Guanidinen und Gemischen hievon ausgewählt ist; und
   (f) Wasser

   besteht, dadurch gekennzeichnet, daß das als Schleifmittel verwendete gefällte Siliciumdioxid 10 bis 300 ppm an Erdalkalimetallionen aufweist und einen RDA-Wert von mindestens 40, einen Ölabsorptionswert von 70 bis 100 $cm^3$/100 g, eine Schüttdichte von 0,24 bis 0,055 $gcm^{-3}$, einen Glühverlust von 4 bis 6 % und eine BET-Oberfläche von 10 bis 300 $m^2$/g mit einer mittleren Teilchengröße von 2 bis 25 $\mu$m, einen Sulfationengehalt unter 0,25 % und einen pH-Wert im Bereich von 4,0 bis 7,5 aufweist.

2. Zahnpastenzusammensetzung nach Anspruch 1, worin das kationische therapeutische Mittel ein substituiertes Guanidin ist.

3. Zahnpastenzusammensetzung nach einem der Ansprüche 1 bis 2, welche zusätzlich eine Zinkionenquelle enthält.

4. Zahnpastenzusammensetzung nach einem der Ansprüche 1 bis 3, worin das grenzflächenaktive Mittel PEG(40)-Sorbitandiisostearat ist.

5. Zahnpastenzusammensetzung nach einem der Ansprüche 1 bis 4, worin das substituierte Biguanidin Chlorhexidindigluconat ist.

6. Zahnpastenzusammensetzung nach Anspruch 3, worin die Zinkionenquelle Zinklactat ist.

7. Zahnpastenzusammensetzung nach einem der Ansprüche 1 bis 6, welche zusätzlich eine Fluoridionenquelle enthält.

8. Zahnpastenzusammensetzung nach einem der Ansprüche 1 bis 7, worin die Fluoridionenquelle Natriumfluorid und das Verdickungsmittel Hydroxyethylcellulose ist.

**Revendications**

1. Composition de dentifrice, essentiellement constituée :
   (a) de 10% à 35% en poids d'une silice précipitée abrasive ayant une bonne compatibilité avec les agents thérapeutiques cationiques ;
   (b) de 0,25% à 2% en poids d'un agent épaississant non ionique choisi parmi l'hydroxyéthylcellulose, l'hydroxypropylcellulose, l'hydroxypropylméthylcellulose, la gomme guar, la gomme de caroube

et des mélanges de ces agents ;

(c) de 0,5% à 2% en poids d'un tensioactif non ionique choisi parmi les copolymères séquencés d'oxyde d'éthylène et d'oxyde de propylène, les esters de sorbitane polyéthoxylés, les esters gras de sorbitane, les acides gras éthoxylés, les esters de PEG et des mélanges de ceux-ci ;

(d) de 30% à 70% en poids d'un humidifiant non ionique choisi parmi le sorbitol, le propylèneglycol, la glycérine et des mélanges de ceux-ci ;

(e) de 0,1% à 5% en poids d'un agent thérapeutique organique cationique choisi parmi les composés d'ammonium quaternaire, les guanidines substituées et des mélanges de ceux-ci ; et

(f) de l'eau,

caractérisée en ce que la silice précipitée abrasive contient 10-300 parties par million d'ions de métal alcalino-terreux et possède une valeur RDA d'au moins 40, un indice d'absorption d'huile de 70-100 $cm^3/100$ g, une masse volumique compactée comprise entre 0,24 et 0,055 $g.cm^{-3}$, une perte au feu comprise entre 4 et 6% et une surface spécifique BET de 10 à 300 $m^2/g$, avec une granulométrie moyenne de 2 à 25 $\mu m$, une proportion d'ions sulfate inférieure à 0,25% et un pH dans le domaine de 4,0-7,5.

**2.** Composition de dentifrice selon la revendication 1, dans laquelle l'agent thérapeutique cationique est une guanidine substituée.

**3.** Composition de dentifrice selon l'une quelconque des revendications 1-2, qui contient en plus une source d'ions zinc.

**4.** Composition de dentifrice selon l'une quelconque des revendications 1-3, dans laquelle le tensioactif est le diisostéarate de PEG (40)-sorbitane.

**5.** Composition de dentifrice selon l'une quelconque des revendications 1-4, dans laquelle le biguanide substitué est le chlorhexidine-digluconate.

**6.** Composition de dentifrice selon la revendication 3, dans laquelle la source d'ions zinc est le lactate de zinc.

**7.** Composition de dentifrice selon l'une quelconque des revendications 1-6, qui contient en plus une source d'ions fluorure.

**8.** Composition de dentifrice selon l'une quelconque des revendications 1-7, dans laquelle la source d'ions fluorure est le fluorure de sodium et l'agent épaississant est l'hydroxyéthylcellulose.